Europäisches Patentamt

**European Patent Office**

**Office européen des brevets**

(11) Publication number: **0 260 350 B1**

## EUROPEAN PATENT SPECIFICATION

(45) Date of publication of patent specification: **12.02.92**  (51) Int. Cl.⁵: **C12N 15/22**, C12P 21/02,
A61K 37/66

(21) Application number: **86306895.3**

(22) Date of filing: **05.09.86**

(54) Oxidation-resistant interferon-beta muteins and their production; formulations containing such muteins.

(43) Date of publication of application:
**23.03.88 Bulletin 88/12**

(45) Publication of the grant of the patent:
**12.02.92 Bulletin 92/07**

(84) Designated Contracting States:
**AT BE CH DE FR GB IT LI LU NL SE**

(56) References cited:
**EP-A- 0 109 748**
**EP-A- 0 130 756**
**EP-A- 0 131 816**
**EP-A- 0 164 719**
**EP-A- 0 169 114**

(73) Proprietor: **CETUS CORPORATION**
**1400 Fifty-Third Street**
**Emeryville California 94608(US)**

(72) Inventor: **Wang, Alice M.**
**2423 Providence Court**
**Walnut Creek California 94596(US)**
Inventor: **Shaked, Ze'ev**
**1038 Sierra Street**
**Berkeley California 94707(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN(GB)**

**Description**

The invention relates to the use of recombinant techniques to provide modified protein sequences resistant to oxidation, i.e., oxidation-receptive proteins. More specifically, the invention relates to providing proteins which retain full biological activity, but which contain conservative amino acid substitutions for specific methionine residues that are particularly susceptible to oxidation.

The fact that different proteins undergo varying degrees of modification during purification is well known, but not well understood. Certain proteins are known to be unusually susceptible to thermal denaturation or to proteolytic cleavage; others contain reactive amino acid side chains located in positions which render them particularly susceptible to chemical modification, including oxidation. In general, it is not possible to predict from the amino acid sequence the extent to which any of the above modifications will occur.

This problem is compounded because ordinary preparative methods for protein purification may lack the resolution to detect or separate small amounts or impurities. Such commonly employed techniques and criteria for homogenicity as electrophoresis, gel filtration and ion exchange chromatography may fail to reveal the presence of small amounts of contaminating material which can be shown to be present by more sensitive analytical techniques, such as reverse phase high performance liquid chromatography (RP-HPLC).

Floor, E., et al., Anal. Biochem. (1980) 101:498-503 describes an illustration of this problem. An undecaprotein which has neurotramsmittor properties, substance P, containing a carboxy terminal methionine that is oxidizable to methionine sulfoxide in dilute solutions exposed to air, elutes from ion exchange columns as a single peak, but reveals the presence of the sulfoxide-containing contaminant when subjected to RP-HPLC. Similarly, Frelinger, A.L., et al., J. Biol. Chem. (1984) 259: 5507-5513 showed that bovine parathyroid hormone which appeared homogenous by conventional criteria could be shown by RP-HPLC analysis to contain sequences with either one or both of the methionine residues oxidized to the sulfoxide. The proteins were eluted from RP-HPLC in an order directly proportional to the number of methionine residues oxidized.

Recently, Rosenberg, S., et al., Nature (1984) 312:77-80 produced a mutagenized human alpha-1 anti-trypsin in yeast which contained a valine residue in place of the methionine at the active site, and which retained significant biological activity. This substitution was made to prevent the observed decrease in inhibitory activity toward elastase, caused by oxidation of this methionine, which destroys the protective function of alpha-1 anti-trypsin in the lungs. The valine substitition resulted in an active anti-trypsin which was, unlike the native form, resistant to oxidation by chemical oxidants which may be similar to those released by leukocytes or present in cigarette smoke. Hence, by making this substitution, it is believed that the levels of anti-trypsin needed to be administered could be reduced. Carrell, R., Nature (1984) 312: 77-80 discloses uses of alpha-antitrypsin mutants such as the Rosenberg et al. mutant in which methionine is replaced by valine. Transgene S.A. has replaced the methionine of alpha-antitrypsin with arginine, presented on May 30, 1985.

When a methionine is located at or near an active site, as in alpha-1 anti-trypsin, and is clearly crucial to biological ac tivity, its oxidation to the sulfoxide might be expected to be detrimental. However, the effects of residual oxidation during purification of proteins containing several methionine residues which are not at the active site is less clear. Data for a number of enzymes containing oxidized methionine residues shows this oxidation to have varying degrees of negative effects on activity. At a minimum, the putatively pure protein obtained will, if the oxidized form is not separated away, contain a contaminant, and will therefore not be homogeneous. The effect of the presence of such contaminant(s) per se in, for example, pharmaceutical protein preparations, is undesirable. Further, the presence of an oxidized methionine may cause other structural changes in the desired protein, such as decreased solubility, increased proteolytic lability, or the inhibition of formation of a required disulfide bond, which may, in turn, have additional side effects, such as the formation of dimeric and higher aggregated forms. Taken together, this collection of impurities may be at least partly responsible for the often observed immunogenicity of parenterally administered proteins, or for other deleterious physiological reactions.

EP 0,130,756 published January 9, 1985 discloses replacing methionine at position 222 of subtilisin with an amino acid such as alanine to provide resistance to oxidation. Estell, D., et al., J. of Biol. Chem. (1985) 260:6518-6521 discloses mutant subtilisin enxaymes whose methionine residues are susceptible to oxidation.

The present invention provides a method for protecting a human therapeutic protein (IFN-Beta) which normally contains methionines from oxidation to the methionine sulfoxide during purification by substituting a conservative amino acid, e.g. alanine, for any methionine particularly susceptible to oxidation. thus, protected, the mutein retains its biological activity, but has enhanced stability. It is not subject to contamination by either modified forms containing the methionine sulfoxide per se, or by side products whose formation is the result thereof.

The invention provides a means to protect specific methionine residues whose preferential oxidation results in therapeutic human protein sequences containing methionine sulfoxide residues. Even in small amounts, these impurities may be potential immunogens, and they detract from the homogenicity of the therapeutic product. Very small amounts of contaminants are significant, especially in the context of drugs injected at high dosage, such as interferons and lymphokines. By providing a conservative amino acid substitution for the susceptible methionine, applicants believe that a majority of the impurities may be prevented.

Thus, in one aspect, the invention relates to a method for protecting a biologically active interferon-beta protein against oxidation comprising making a conservative amino acid substitution for each methionine residue susceptible to chloramine T or peroxide oxidation, where other methionine residues in the reference protein which are significantly less susceptible are not so substituted. The resulting oxidation-resistant mutein has substantially the same activity as the reference protein (i.e, it has the same qualitative biological effect) but is capable of resisting low level oxidation commonly encountered in purification of storage so as to provide a homogenous preparation by the criterion of RP-HPLC and other analytical techniques which detect methionine sulfoxide. In other aspects, the invention relates to the muteins so produced, recombinant DNA sequences encoding the muteins, recombinant expression vectors comprising the DNA sequence operably linked to control sequences compatible with a suitable host, host cells transformed with the vector, and therapeutic formulations containing certain of the muteins so produced.

In another aspect, the invention relates to IFN-Beta muteins which harbor con servative amino acid substitutions in position 62 and to therapeutic formulations containing such muteins.

BRIEF DESCRIPTION OF THE DRAWING

The Figure shows the amino acid sequence of native beta-interferon (IFN-Beta), and the position number designations used herein.

A. Definitions

As used herein, "reference" protein refers to IFN-Beta of known amino acid sequence and whose biological activity is also known, which protein contains at least one methionine which becomes oxidized upon treatment with chloramine T. It has been shown that chloramine T preferentially oxidizes, in native (non-denatured) proteins, only methionine residues which are exposed (Shechter, Y., et al., Biochem. 14:4497-4503). Treatment of peptides or of a denatured native protein, in at least some cases, results in oxidation of all methionine residues to the sulfoxides. Oxidation of any available sulfhydryl groups to disulfide linkages may also occur. Treatment with hydrogen peroxide under appropriate conditions also results in conversion of methionine to methionine sulfoxide.

"Oxidation-resistant mutein", in relation to the "reference" protein, refers to a related amino acid sequence which has a biological activity substantially the same as the reference protein. However, it contains a conservative amino acid substitution for any methionine residue which is particularly susceptible to oxidation by chloramine T or hydrogen peroxide. Such substitutions are not intended to render the mutein resistant to all oxidations, such as beneficial oxidations which might include creation of a disulfide linkage required for full activity.

A methionine "susceptible to oxidation by chloramine T or peroxide" refers to a methionine which undergoes relatively rapid, i.e., detectable, conversion to the sulfoxide in the presence of either of these reagents under the conditions specified below. For treatment with either reagent the protein is dissolved in buffer, 50 mM Tris-HCl, pH 8.0, for chloramine T, 50 mM sodium phosphate, pH 6.8 for peroxide, at a concentration of about 0.2 mg/ml. For some recombinantly produced proteins, solubilizing agents, e.g., 0.1% detergent, may be required to effect solubility. For chloramine T, the oxidant is added to a two-fold molar excess, and the reaction incubated for 15 minutes at 25$^{\circ}$C. For peroxide, $H_2O_2$ is added to 30 mM and the reaction incubated for one hour at 25$^{\circ}$C. Chloramine T oxidation and hydrogen peroxide oxidation exhibit overlapping specificities with respect to methionine. Significant (detectable) oxidation with respect to either of these reagents under the above specified conditions defines a methionine residue as "susceptible" for purposes herein.

A "non-susceptible" methionine residue is defined as a methionine residue which is oxidizable, but is not oxidized detectably (by the means used to detect susceptible methionines) under the conditions specified above, i.e., it is oxidized at a slower rate than the "susceptible" methionine residues. The methionines in proteins have variable susceptibility to oxidation, and the preferred compounds have the most susceptible methionine(s) replaced. The degree of susceptibility and the number of methionines to be replaced will depend on the protein. If it is possible to make the protein homogeneous without further methionine substitutions, then only the most susceptible methionine(s) will be replaced because it is desirable to keep the protein sequence as close to the native protein sequence as possible.

A "conservative" amino acid alteration is defined as one which does not adversely affect biological activity. Conservative amino acid substitutions for oxidizable methionines, according to the invention, are selected from neutral or non-polar amino acids. Preferred are glycine, alanine, serine, threonine, valine, isoleucine, leucine, asparagine, glutamine , glutamate, tyrosine, and phenylalanine. More preferred are alanine, serine, threonine, valine, leucine, and isoleucine. Even more preferred are alanine, serine, leucine, glutamate, and valine, and most preferred in alanine. Also included among conservative alterations is deletion of methionine.

The relationship, therefore, of the "reference" protein to the oxidation -resistant mutein is that of identical amino acid sequence except for substitution of a conservative amino acid for the susceptible methionine, or the deletion thereof.

"Recombinant host cells", "host cells", "cells", "cell cultures", and so forth are used interchangeably, and designate individual cells, cell lines, cell cultures and harvested cells which have been, or are intended to be, transformed with the recombinant vectors of the invention. These terms also include the progeny of the cells originally receiving the vector. It is well understood that not all of the progeny of a single cell are precisely, necessarily identical with the parent, due to spontaneous or intentional mutations or alterations in the culture conditions. These progeny are also included in the definition, so long as the capacity to perform the function of producing the oxidation-resistant mutein of the invention conferred by the vector is retained.

"Transformed" refers to any process for altering the DNA content of the host, including in vitro transformation procedures as described below, phage infection, or such other means for effecting controlled DNA uptake as are known in the art.

"Operably linked" as used herein regarding DNA sequences or genes refers to the situation wherein the sequences or genes are juxtaposed in such a manner as to permit their ordinary functionality. For example, a promoter operably linked to a coding sequence refers to those linkages where the promoter is capable of controlling the expression of the sequence.

"Control sequences" refers to DNA sequences which control the expression of the sequence which encodes the oxidation-resistant mutein. Examples include promoters for transcription initiation, optionally with an operator, enhancer regions, ribosome binding site sequences, and translation signals which initiate and terminate translation of the gene. Such control sequences must be compatible with, i.e., operable in, the host into which they will be inserted.

"Inert, non-allergenic, pharmaceutically compatible carrier" refers to a carrier for the mutein herein which does not react with the mutein, is water soluble and preferably non-sensitive to water, is itself stable, does not elicit an allergenic response in a patient, and is compatible with the mutein physiologically and pharmaceutically so that a stable, soluble formulation is made. The carrier may be liquid or solid and if solid, may be a solid bulking agent for pharmaceutical tablet formulation. The invention embraces both pharmaceutical and veterinary therapeutic formulations containing the present new muteins. Such formulations may be made in accordance with standard practice and may be in unit dosage form.

B. General Description

To obtain oxidation resistant muteins of the invention, a reference IFN-Beta protein is identified and the specific methionine residue which exhibits preferential oxidation is replaced by a conservative amino acid substitution.

RP-HPLC in some instances will be adequate to resolve reference protein from reference protein which contains methionine sulfoxide. The protein is subjected to analysis on RP-HPLC before and after treating with, e.g., chloramine T under the specific conditions set form in A above. (Alternatively, peroxide could be used.) Conditions are selected whereby only the most susceptible methionine residues are oxidized to the sulfoxide. Both the major peak resulting from chloramine T oxidation, and the major peak obtained without such oxidation, are subjected to procedures designed to determine the position of any methionine residue which has undergone oxidation.

A variety of procedures is available. However, a particularly convenient procedure utilizes cyanogen bromide cleavage (which reagent cleaves at methionine residues, but is unreactive when those methionine residues are oxidized), followed by HPLC peptide mapping and sequence analysis.

The cyanogen bromide cleavage mixture is first reduced with, for example, dithioerythritol to destroy any disulfide linkages remaining. For the reference protein, untreated with chloramine T, the number of peptides obtained should equal the number of internal methionine residues plus 1. For the protein oxidized by chloramine T, the number of resulting peptides will be diminished in proportion to the number of internal methionines oxidized. Thus, a comparison of the number of peptides obtained upon treatment by cyanogen bromide cleavage, DTE reduction, and HPLC analysis will permit a conclusion as to the number of internal methionine residues present, and the number which are exposed for oxidation by chloramine T. Sequencing of the peptides thus obtained, along with, for example, comparison to the complete amino acid sequence independently established, will then reveal the location of the oxidized methionine with respect to the complete sequence.

After the location of the susceptible methionine is established, the coding sequence for the protein is used for site-specific mutagenesis. the coding sequence for interferon-beta is already known and available in the art.

To prepare an oxidation-resistant mutein of the reference IFN-Beta protein, a DNA sequence encoding the reference protein, cloned into a convenient M-13 cloning vector, is subjected to site-specific mutagenesis using the appropriate primer to convert the residue at the identified position from methionine to a conservative amino acid replacement. Site-specific (or primer-directed) mutagenesis is now a technique which is well-established in the art. Briefly, a synthetic oligonucleotide complementary to the desired sequence is used as a primer in the synthesis of a strand complementary to the phage single-stranded reference sequence. The resulting double-stranded DNA is transformed into a phage-supporting host bacterium. Cultures of the transformed bacteria are plated in top agar, permitting plaque formation from single cells which harbor the phage. Theorectically, 50% of the plaques will consist of phage containing the mutated form; 50% will have the original sequence. The plaques are hybridized with kinased synthetic primer under stringency conditions which permit hybridization only with the desired sequence, which will form a perfect match with the probe. Hybridizing plaques are then picked and cultured, and the DNA is recovered.

The resulting DNA is then ligated into expression vectors using standard procedures, which can be precisely identical to those used in preparing expression vectors for the reference sequence. An oxidation-resistant mutein may then be produced in suitable hosts under the control of compatible control sequences using any of the recombinant host cell systems known in the art (see C.1). The methionine-replaced muteins thus produced are recovered and purified using standard protein purification techniques.

One protein purification technique if the protein is in the form of a refractile material produced from a microorganism host is to (a) disrupt the cell membrane of the host, (b) remove greater than 99% by weight of the salts from the disruptate, (c) redisrupt the desalted disruptate, (d) add a material to the disruptate such as sucrose to increase the density or viscosity of, or to create a density or viscosity gradient in, the liquid within the disruptate, and (e) separate the refractile material from the cellular debris by high speed centrifugation of 10,000 to 40,000 x g. Preferably the sucrose is added to increase the density o f the liquid to a P of between 1.13 and 1.17 $g/cm^3$.

In a further embodiment, the first alternative is employed to isolate the refractile bodies and then the refractile bodies are dissolved in a solubilizing agent such as SDS in the presence of a reducing agent and reduced material is separated, oxidized and purified by gel filtration or reverse-phase high performance liquid chromatography.

Oxidation IFN-Beta during the recovery step may be carried out by using iodosobenzoic acid, as described by U.S. Patent No. 4,530,787, or by using a copper ion, such as copper chloride at a pH of about 5.5 and 9.

The oxidation-resistant preparations have similar biological activity to that of the pertinent reference protein, and therefore will be in the same applications.

B.1. IFN-Beta Oxidation-Resistant Muteins

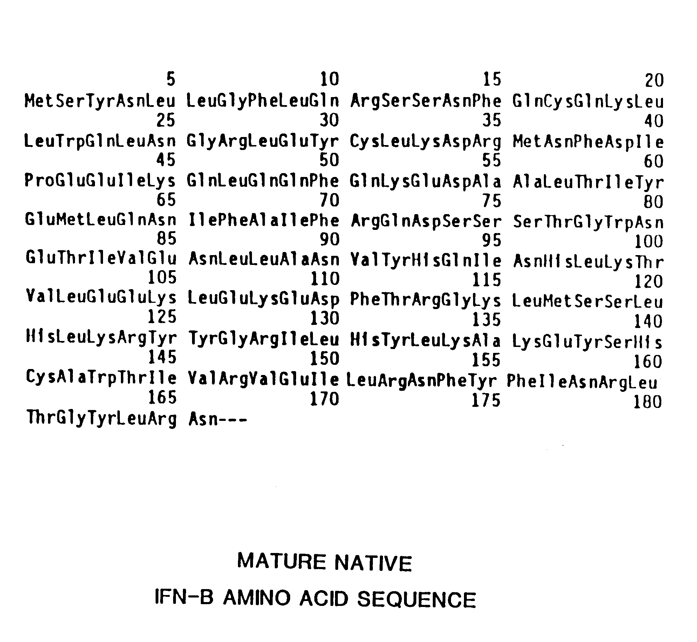

The 166 amino acid sequence of native IFN-Beta is shown in the Figure. There are three cysteine residues, at positions 17, 31 and 141. The cysteine residue at position 17 has been shown not to be essential for biological activity, and IFN-Beta muteins with conservative amino acid substitutions at position 17 have been disclosed in US Patent No. 4,518,584 issued May 21, l985, and incorporated herein by reference. It has been concluded that the disulfide bridge found in both the native and mutated forms of IFN-Beta is between the cysteine at position 141 and at position 31. In addition, the IFN-Beta sequence, as shown in Figure 1, contains four methionine residues (at positions 1, 36, 62 and 117).

Forms of IFN-Beta prepared for clinical testing have been shown to contain minor amounts of contaminants using analytical techniques with higher resolution than the preparative purification procedures used to manufacture the clinical samples. Both for proteins in general, and for IFN-Beta in particular, one cause for production of contaminants during purification, due to alteration of three-dimensional structure or formation of complexes, resides in the ability of certain free sulfhydryl groups to form disulfides other than those present in the active form of protein. Accordingly, purification procedures for IFN-Beta are conducted under conditions which maintain cysteine residues in the sulfhydryl condition prior to permitting disulfide bond formation under controlled conditions of moderate oxidation. Nevertheless, it has now been found that because equipment and reagents used in purification procedures for pharmaceutical proteins must be sterile, residual oxidants, such as hydrogen peroxide, from sterilizing wash solutions may be present in sufficient amounts to cause oxidative conversions in the IFN-Beta preparations. Furthermore, such oxidations may also occur on storage.

All of the above-mentioned forms of IFN-Beta may be used as the reference protein which is designed to delete or substitute a conservative amino acid for the methionine residue susceptible to such oxidation. Oxidation-resistant muteins may be valuable in providing compositions which retain potency as protein products, or oxidation resistance may alter the pharmacological behavior of the molecules in other beneficial ways.

Purification procedures performed on $ser_{17}$ IFN-Beta result in products which give similar patterns on RP-HPLC analysis to those obtained from purified clinical product IL-2. Oxidation of IFN-Beta with hydrogen peroxide, using conditions described in section A, has the effect of eliminating the HPLC peak associated with methionine sulfoxide formation (i.e., oxidized methionine). The biological activity of IFN-Beta where at least the methionine at position 62 is oxidized is 1-5% of the activity of the non-oxidized counterpart. The mutein forms of IFN-Beta with a methionine at position 36, 62 or 117 replaced may be obtained through site-specific mutagenesis. The muteins are then oxidized and the HPLC results are analyzed for the presence of the peack characteristic of the oxidized form to deter mine the location of the affected methionine.

After site-specific mutagenesis, an expression clone is prepared containing a promoter, the IFN-Beta mutein-encoding gene, and an appropriate expression vector. Suitable cells are transformed with the clone and grown in a fermentation medium or in a shaker flask.

The interferon-beta mutein is recovered from the culture in which the transformed cells are gown by disrupting the cells, typically by homogenization or sonication. The cells may be concentrated prior to disruption, by, for example, diafiltration.

The cellular debris is then treated with one or more solubilizing agents such as SDS and dithiothreitol for a period of about one hour. The resulting cell suspension is then extracted with one or more extractants, such as 2-butanol, to isolate the crude interferon-beta, usually using a 1:1 2-butanol:suspension volume ratio in a static mixer as described in US Patent 4,450,,103 issued May 22, 1984. The mixture is then centrifuged and the extractant-rich, organic phase is collected.

To the organic phase, adjusted to a pH of about 6-8, preferably about 6.2, is added a precipitating solution containing salts and, for example, SDS. The resulting suspension is centrifuged and the interferon-rich pellet is suspended in a solution, e.g., containing SDS. Then an oxidizing agent such as o-iodosobenzoic acid or copper chloride is added as described in US Patent Nos. 4,530,787 and 4,572,798, respectively.

After the oxidation, the reaction mixture is placed on a preparative RP-HPLC column to purify, and then on an analytical RP-HPLC column to purify further and to characterize the product.

The interferon can then be assayed for antiviral activity using the yield reduction assay described by Steward and Lockhart, J. of Virol., 6:795-799 (1970) or the cytopathic effect assay described by Steward, W.E., II, The Interferon System (New York:Springer-Verlag, 1981), p.17.

The interferon may be formulated in the same manner as native IFN-Beta. For therapeutic or diagnostic applications, it may be formulated in non-toxic, non-allergenic, pharmaceutically compatible carrier media such as distiller water, Ringer's solution, Hank's solution of physiological saline. The formulation may also contain non-toxic stabilizers such as dextrose and non-toxic stabilizers such as albumin. Administration of the IFN-Beta muteins to humans or animals may be, for example, intravenous, intraperitoneal, intramuscular or subcutaneous as deemed appropriate by the physician. The amount of IFN-Beta mutein administered will usually range between about $1 \times 10^4$ and $2 \times 10^8$ units, depending on the subject and its weight.

As indicated, the IFN-Beta may be formulated in a non-toxic, non-allergenic, pharmaceutically compatible carrier medium such as distilled water, Ringer's solution, Hank's solution, or physiological saline. A water-soluble carrier may be added to the desired level. The carrier will typically be added such that it is present in the solution at about 1 to 10% by weight, preferably about 5% by weight. The exact amount of carrier added is not critical. Conventional solid bulking agents which are used in pharmaceutical table formulation may be used as the carrier. These materials are water soluble, do not react with the IFN-Beta,

6

and are themselves stable. They are also preferably non-sensitive (i.e. non-hygroscopic) to water. Examples of carriers that may be added are non-toxic stabilizers such as mannitol or other materials such as lactose and other reduced sugars such as sorbitol, starches and starch hydrolysates derived from wheat, corn, rice, and potato, microcrystalline celluloses, and albumin such as human serum albumin.

The carrier adds bulk to the formulation such that when unit dosage amounts of the solution are lyophilized in containers, such as sterile vials, the freeze-dried residue will be clearly discernible to the naked eye. In this regard the preferred carrier, mannitol, yields an aesthetically acceptable (white, crystalline) residue which is not sensitive to water. The nonsensitivity of mannitol to water may enhance the stability of the formulation.

After the carrier is added, the unit dosage amounts of the solution are dispensed into containers, the containers are capped with a slotted stopper, and the contents are lyophilized using conventional freeze-drying conditions and apparatus.

The lyophilized, sterile product consists of a mixture of (1) recombinant IFN-Beta, (2) carrier (mannitol), (3) detergent (SDS), and (4) a small amount of buffer which will provide a physiological pH when the mixture is reconstituted.

The lyopholized mixture may be reconstituted by injecting a conventional parenteral aqueous injection such as water for injection, Ringer's injection, dextrose injection, dextrose and salt injection, or the like, into the vial. The injection should be added against the side of the vial to avoid excess foaming.

Thus, the IFN-Beta muteins may be formulated as described, including modification by means of an activated homopolymer such as polyethylene glycol.

The IFN-Beta muteins are useful as anti-viral, anti-psoriatic, anti-proliferative, immunomodulatory and anti-tumor agents. When purified and formulated as described above, the IFN-Beta mutein with alanine substituted at position 62 has 4% of the biological activity of the native-sequence IFN-Beta. Other amino acid substitutions at position 62 may be expected to have different levels of IFN-Beta activity.

C. Standard Methods

Most of the techniques which are used to tranform cells, construct vectors, effect hybridisation with probes, and the like are widely practiced in the art, and most practitioners are familiar with the standard resource materials which describe specific conditions and procedures. However, for convenience, the following paragraphs may serve as a guideline.

C.1. Hosts and Control Sequences

Either procaryotic or eucaryotic hosts may be used for expression of DNA sequences; cloning of such sequences generally employs procaryotes for convenience. Procaryotes most frequently are represented by various strains of E. coli. However, other microbial strains may also be used, such as bacilli, for example, Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid from an E. coli species by Bolivar, et al., Gene (1977) 2:95. pBR322 contains genes for ampicillin and tetracycline resistance, and thus provides additional markers which can be either retained or destroyed in constructing the desired vector. Commonly used procaryotic control sequences include such commonly used promoters as the beta-lactamase (penicillinase) and lactose (lac) promoter systems (Chang, et al., Nature (1977) 198:1056) and the tryptophan (trp) promoter system (Goeddel, et al., Nucleic Acids Res. - (1980) 8:4057) and the lambda derived P $_L$ promoter and N-gene ribosome binding site (Shimatake, et al., Nature (1981) 292:128). Any available promoter system compatible with procaryotes can be used.

In addition to bacteria, eucaryotic microbes, such as yeast, may also be used as hosts. Laboratory strains of Saccharomyces cerevisiae, Baker's yeast, are most used, although a number of other strains are commonly available. Plasmid vectors suitable for yeast expr ession include use of the 2 micron origin of replication (Broach, J. R., Meth. Enz. (1983) 101:307), and those described by, for example, Stinchcomb, et al., Nature (1979) 282:39, Tschempe, et al., Gene (1980) 10:157 and Clarke, L., et al., Meth. Enz. (1983) 101:300). Control sequences for yeast vectors include promoters for the synthesis of glycolytic enzymes (Hess, et. al., J. Adv. Enzyme Reg. (1968) 7:149; Holland, et al., Biochemistry (1978) 17:4900). Additional promoters known in the art include the promoter for 3-phosphoglycerate kinase (Hitzeman, et al., J. Biol. Chem. (1980) 255:2073), as well as those for other glycolytic enzymes. Other promoters are available which have the additional advantage of transcription controlled by growth conditions such as the promoter regions for alcohol dehydrogenase 2, isocytochrome C, acid phosphatase, degradative enzymes associated with

7

nitrogen metabolism, and enzymes responsible for maltose and galactose utilization (Holland, ibid). It is also believed that terminator sequences are desirable at the 3′ end of the coding sequences. Such terminators are found in the 3′ untranslated region following the coding sequences in yeast-derived genes such as those derived from the enolase gene containing plasmid peno46 (Holland, M. J., et al., J. Biol. Chem. (1981) 256:1385) or the LEU2 gene obtained from YEp13 (Broach, J., et al., Gene (1978) 8:121).

It is also, of course, possible to express genes encoding polypeptides in non-human eucaryotic host cell cultures derived from multicellular organisms. See, for example, Tissue Cultures, Academic Press, Cruz and Patterson, editors (1973). Useful host cell lines include VERO, HeLa cells, Chinese hamster ovary (CHO) cells, as well as fungal systems such as Aspergillus. Expression vectors for such cells ordinarily include promoters and control sequences compatible with mammalian cells such as, for example, the commonly used early and late promoters from Simian Virus 40 (SV 40) (Fiers, et al., Nature (1978) 273:113), or other viral promoters such as those derived from polyoma, Adenovirus 2, bovine papiloma virus, or avian sarcoma viruses. General aspects of mammalian cell host system transformations have been described by Axel; U.S. Patent No. 4,399,216 issued August 16, 1983. It now appears, also that "enhancer" regions are important in optimizing expression; these are, generally, sequences found upstream or downstream of the promoter region in non-coding DNA regions. Origins of replication may be obtained, if needed, from viral sources. However, integration into the chromosome is a common mechanism for DNA replication in eucaryotes. Plant cells are also now available as hosts, and control sequences compatible with plant cells such as the nopaline synthase promoter and polyadenylation signal sequences (Depicker, A., et al., J. Mol. Appl. Gen. (1982) 1:561) are available.

C.2. Transformations

Depending on the host cell used, transformation is done using standard techniques appropriate to such cells. The calcium treatment employing calcium chloride, as described by Cohen, S. N., Proc. Natl. Acad. Sci. (USA) (1972) 69:2110, or the RbCl$_2$ method described in Maniatis, et al., Molecular Cloning: A Laboratory Manual (1982) Cold Spring Harbor Press, p. 254 is useful for procaryotes or other cells which contain substantial cell wall barriers. Infection with Agrobacterium tumefaciens (Shaw, C. H., et al., Gene - (1983) 23:315) is used for certain plant cells. For mammalian cells without such cell walls, t he calcium phosphate precipitation method of Graham and van der Eb, Virology(1978) 52:546 is preferred. Transformations into yeast are carried out according to the method of Van Solingen, P., et al., J. Bact. (1977) 130:946, Hsiao, C. L., et al., Proc. Natl. Acad. Sci. (USA)(1979) 76:3829, and Klebe, R. J. et al., Gene (1983) 25:333.

C.3 Probing cDNA or Genomic Libraries

cDNA or genomic libraries are screened using the colony hybridization procedure. Each microtiter plate is replicated onto duplicate nitrocellulose filter papers (S & S type BA-85) and colonies are allowed to grow at 37°C for 14-16 hours on L agar containing 50 μg/ml Amp. The colonies are lysed and DNA is fixed to the filter by sequential treatment for five minutes with 500 mM NaOH, 1.5 M NaCl. The filters are washed twice for five minutes each time with 5 x standard saline citrate (SSC). Filters are air dried and baked at 80°C for two hours. The duplicate filters are prehybridized at the desired temperature for a desired time period, with 10 ml per filter of DNA hybridization buffer (5 x SSC, pH 7.0 5 x Denhardt's solution (polyvinylpyrrolidine, plus Ficoll and bovine serum albumin; 1 x = 0.02% of each), 50 mM sodium phosphate buffer at pH 7.0, 0.2% SDS, 20 μg/ml poly U, and 50 μg/ml denatured salmon sperm DNA).

The samples are hybridized with kinased probe under conditions which depend on the stringency desired. Typical moderately stringent conditions employ a temperature of 42°C for 24-36 hours with 1-5 ml/filter of DNA hybridization buffer containing probe. For higher stringencies higher temperatures and shorter times are employed; for lower stringencies, the reverse. The filters are washed four times for 30 minutes each time at 37°C with 2 x SSC, 0.2% SDS and 50 mM sodium phosphate buffer at pH 7, then are washed twice with 2 x SSC and 0.2% SDS, air dried, and are autoradiographed at -70°C for two to three days.

C.4 Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site-specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs Product Catalog. In general, about 1 $\mu$g of plasmid or DNA sequence is cleaved by one unit of enzyme in about 20$\mu$l of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separations is found in Methods in Enzymology (1980) 65:499-560.

Restriction-cleaved fragments may be blunt ended by treating with the large fragment of E. coli DNA polymerase I (Klenow) in the presence of the four deoxynucleotide triphosphates (dNTPs) using incubation times of about 15 to 25 minutes at 20 to 25°C in 50 mM Tris pH 7.6, 50 mM NaCl, 6 mM MgCl$_2$, 6 mM DTT and 5-10 $\mu$M dNTPs. Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column. Treatment under appropriate conditions with Sl nuclease results in hydrolysis of any single-stranded portion.

Synthetic oligonucleotides are prepared by the triester method of Matteucci, et al., ( J. Am. Chem. Soc. (1981) 103:3185) or using commercially available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM MgCl$_2$, 5 mM dithiothreitol, 1-2 mM ATP, 1.7 pmoles $\gamma$32P-ATP (2.9 mCi/mmole), 0.1 mM spermidine, 0.1 mM EDTA.

Ligations are performed in 15-30 $\mu$l volumes under the following standard conditions and temperatures: 20 mM Tris-Cl pH 7.5, 10 mM MgCl$_2$, 10 mM DTT, 33 $\mu$g/ml BSA, 10 mM-50 NaCl, and either 40 $\mu$M ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 $\mu$g/ml total DNA concentrations (5-100 nM total end concentration). Intermolecular blunt end ligations (usually employing a 10-30 fold molar excess of linkers) are performed at 1 $\mu$M total ends concentration.

In vector construction employing "vector fragments", the vector fragment is commonly treated with bacterial alkaline phosphatase (BAP) in order to remove the 5' phosphate and prevent religation of the vector. BAP digestions are conducted at pH 8 in approximately 150 mM Tris, in the presence of Na$^+$ and Mg$^{+2}$ using about 1 unit of BAP per $\mu$g of vector at 60°C for about one hour. In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

C.5 Verification of Construction

In the constructions set forth below, correct ligations for plasmid construction are confirmed by first transforming E. coli strain MM294 obtained from E. coli Genetic Stock Center, CGSC #6135, or other suitable host, with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al., Proc. Natl. Acad. Sci. (USA) (1969) 62:1159, optionally following chloramphenicol amplification (Clewell, D. B., J. Bacteriol. (1972) 110:667). The isolated DNA is analyzed by restriction and/or sequenced by the dideoxy method of Sanger, F., et al., Proc. Natl. Acad. Sci. (USA) (1977) 74:5463 as further described by Messing, et al., Nucleic Acids Res. (1981) 9:309, or by the method of Maxam, et al., Methods in Enzymology (1980) 65:499.

C.6 Hosts Exemplified

Host strains used in cloning and expression herein are as follows:
For cloning and sequencing, and for expression of construction under control of most bacterial promoters, E. coli strain MM294 (supra), Talmadge, K., et al., Gene (1980) 12:235; Meselson, M., et al.,

Nature (1968) 217:1110, was used as the host.

For M13 phage recombinants, E. coli strains susceptible to phage infection, such as E. coli K12, DG98, are employed. The DG98 strain has been deposited with ATCC on July 13, 1984 and has accession number 39,768. For yeast transformations, S. cerevisiae strains such as C468 (Innis, M. A. et al., Science - (1985) 228:21-26 and its cir °derivative were used. C468 cir ° was deposited with ATCC on December 13, 1985 and has ATCC accession no. 20,787. For expression of IFN-$\beta$ muteins in E. coli, strains may be used such as MM294, which has been deposited with ATCC on February 14, 1984 and has ATCC accession number 39,607.

In the examples which follow, all temperatures are provided in degrees Celsius and all parts and percentages are by weight, unless otherwise indicated. These examples are meant only to typify the procedure, and are not to be construed as limiting. Therefore, other reference forms of IFN-$\beta$ may be used, and other expression systems may be used to provide the protein. For example, a yeast construct can be designed to result in an intracellular yeast product lacking the leader sequence described herein. As stated above, desired coding sequences may be expressed in a variety of procaryotic and eucaryotic host systems using known control sequences.

## EXAMPLE

### D. PREPARATION OF OXIDATION-RESISTANT MUTEINS OF IFN-BETA

Recombinant $ser_{17}$IFN-Beta containing a methionine residue at position 62 may be modified to produce muteins such as $ser_{17}ala_{62}$IFN-Beta. The construction of the potentially oxidation-resistant mutein $ser_{17}ala_{62}$IFN-Beta is described.

### Preparation of Coding Sequence

A plaque designated as M13-SY2501 containing the coding sequence for $ser_{17}$IFN-Beta was prepared as described in Mark, D.F. et al. (1984) Proc. Natl. Acad. Sci. USA 81:5662-5666. This clone M13-SY2501 was used as a template for oligonucleotide-directed mutagenesis to convert the methionine at position 62 to an alanine residue. 40 pmoles of the oligonucleotide 5'-CCATCTATGAGGCGCTGCAGAACATC-3' was kinased under standard conditions for use as primer and probe. Ten pmoles of the kinased primer was hybridized to 2.5 ug of single-stranded (SS) M13-SY2501 in 15 ul of a mixture containing 100 mM NaCl, 20 mM Tris-HCl, pH 7.9, 20 mM MgCl$_2$, and 20 mM Beta-mercaptoethanol by heating at 67°C for five minutes and 42°C for 25 minutes. The annealed mixture was chilled on ice and adjusted to a final volume of 25 ul in a reaction mixture containing 0.5 mM of each dNTP, 17 mM Tris-HCl, pH 7.9, 17 mM MgCl$_2$, 83 mM NaCl, 17 mM Beta-mercaptoethanol, 5 units of DNA polymerase I, Klenow fragment, 0.5 mM ATP, and 2 units of T$_4$ DNA ligase, incubated at 37°C for 30 units of T$_4$ DNA ligase, incubated at 37°C for 30 minutes. the reactions were terminated by heating to 70°. The reaction mixtures were used to transform competent E. coli K12 DG 98 cells, which were plated onto agar plates, and incubated overnight to obtain phage plaques. DG 98 cells are described more fully in co-pending EP Publication No. 137,280 published April 17, 1985, and were deposited with the American Type Culture Collection, Rockville, MD on July 13, 1984 under ATCC No. 39, 768. The plaques were probed using kinased primer using standard prehybridization and hybridization conditions at high stringency (60°C for two hours). A plaque which hybridized to primer was selected. This plaque was designated M13-DM101 and contains the coding sequence for $ser_{17}ala_{62}$IFN-Beta.

### Construction of Expression Clone

To construct the host vector containing the trp control behind a HindIII site, the trp promoter/operator/ribosome binding site sequence, lacking the attenuator region, was derived from pVH153, obtained from C. Yanofsky, Stanford University, Stanford, CA. Trp sequences are available in a variety of plasmids well known in the art. pVH153 was treated with HhaI (which cuts, leaving an exposed 3' sticky end just 5' of the trp promoter), blunt-ended with Klenow, and partially digested with TaqI. The 99 pb fragment corresponding to restriction at the TaqI site, six nucleotides preceding the ATG start codon of trp leader, was isolated, and then ligated to EcoRI (repair)/ ClaI digested, pBR322 to provide pTRP3. pTRP3 was deposited on December 18, 1984 and has ATCC accession number 39,946.

RF-DNA from M13-DM101 was digested with HindIII and XhoII, and the fragment containing the mutagenized IFN-Beta coding sequence was purified from a 1% agarose gel and inserted into the HindIII and BamHI sites of plasmid pTRP3, described above, containing the trp promoter. The ligation mixture was transformed into competent E. coli K12 strain MM294, generating an ampicillin-resistant phenotype. The resulting clone, designated pAW207, containing the coding sequence for $ser_{17}ala_{62}$IFN-Beta, was confirmed by restriction analysis and dideoxy sequencing. This clone was deposited with the American Type Culture Collection (ATCC), 12301 Parklawn Drive, Rockville, MD under ATCC No. 67,138 on June 19, 1986.

Production and Purification of $ser_{17}Ala_{62}$-IFN-Beta

For growth of E. coli tranformed with this expression clone, the following basal medium was used:
$NH_4Cl$ 10 mM
$KH_2PO_4$ 21.9 mM
$Na_2HPO_4$ 28.1 mM
$K_2SO_4$ 9 mM
$MgSO_4$ 2 mM
$MnSO_4$ $3\mu M$
$ZnSO_4$ 3 $\mu M$
$CuSO_4$ 0.1 $\mu M$
$FeSO_4$ 10 $\mu M$
Glucose 2 g/l
Ampicillin 50 mg/l

An overnight culture was grown at 37° C in the above medium supplemented with 50 mg/l L-tryptophan. The cells were washed once in basal medium and resuspended in basal medium supplemented with casamino acids (20 g/l) and Beta-indole acrylic acid (10 mg/l) to an optical density of 0.2 (680 nm). The culture was shaken at 37° C for four hours, and the cells were harvested by centrifugation. Approximately 300 mg dry weight cells was derived from a 750 ml culture by centrifugation.

The cells were then resuspended in 2% SDS in phosphate buffered saline (PBS) (consisting of 0.15 M NaCl, 0.0175 M $NaPO_4$, and 0.01475 M NaOH) and disrupted by sonication. Solid dithiothreitol (DTT) was then added to a final concentration of 10 mM and the homogenate was treated to 50 ± 5° C for 10 minutes. The resulting solubilized cell disruptate was extracted with 2-butanol and 2 mM dithiothreitol (DTT) at 1:1 2-butanol/DTT:suspension volume ratio in a static mixer. The mixture was then centrifuged and the 2-butanol-rich phase was collected.

The 2-butanol-rich phase was mixed with 2.5 volumes of 0.1% w/v SDS in PBS. The pH of the mixture was adjusted to 6.2 ± 0.1 with glacial acetic acid and this mixture was centrifuged. The resulting paste was collected and resuspended in a mixture of 50 mM $NaHPO_4$, 5 mM EDTA and 5% SDS with pH adjustment to 8.5 ± 0.1 using 1 N NaOH. Solid DTT was added to a final concentration of 10 mM and the suspension was heated to 50 ± 5° C for 10 minutes. The suspension was then cooled to about 25° C, the pH was adjusted to 5.5 ± 0.1 with glacial acetic acid, and the solution was filtered.

The solution was then applied to a Sephadex G-25 desalting column which was equilibriated with 50 mM sodium pho sphate, 0.1% SDS, pH 7.0. The peak, $A_{280}$-absorbing fraction was pooled.

The protein was oxidized to generate disulfide bonds. The reaction mixture contained approximately 0.25 mg/ml of the interferon. Oxidation was initiated by adding $CuCl_2$ to a final concentration of 1 mM at 25° C.

The oxidized product was applied to a 1.3 cm Vydac ™ $C_4$ preparative RP-HPLC column in a water:-acetonitrile gradient in 0.1% v/v trifluoroacetic acid to separate the oxidized interferon from the E. coli contaminant proteins. The IFN peak was pooled and the pool was subjected to an analytical RP-HPLC in the same gradient.

Activity of Mutein

When tested for antivial activity using the yield reduction assay as described by Steward and Lockhart, supra, the oxidized protein was found to have 13% of the activity of the $ser_{17}$IFN-Beta mutein described in US Patent No. 4,588,585 issued May 13, 1986.

Homogeneity of the Mutein

When the mutein was subjected to the analytical RP-HPLC, a single symmetrical peak was obtained, showing that the peak A impurity material which was observed in the RP-HPLC of similarly purified $ser_{17}$IFN-Beta, $ser_{17}ala_{36}$IFN-Beta, and $ser_{17}ala_{117}$IFN-Beta has been eliminated. (The correlation of peak A with an oxidized methionine in $ser_{17}$IFN-Beta was confirmed by oxidizing the $ser_{17}$IFN-Beta sample with hydrogen peroxide and comparing the pre-oxidation and post-oxidation RP-HPLC.)

The mutein was then oxidized with 0.5% hydrogen peroxide at pH 5.0 and then analyzed by analytical RP-HPLC. No change in the RP-HPLC pattern was obtained for the oxidized mutein, indicating that this mutein lacks the methionine residue preferentially susceptible to oxidation to Peak A by hydrogen peroxide. The main peak for the oxidized $ser_{17}ala_{62}$IFN-Beta mutein was, however, shifted in the RP-HPLC compared to the main peak for the unoxidized form, indicating that perhaps other unsubstituted methionines or other amino acids in the mutein were being oxidized to some extent, but not to a species corresponding to Peak A.

At least any one of the first five amino acids may be deleted, in any combination, or substituted by another amino acid, at the N-terminus of the corresponding oxidation-resistant IL-2 muteins with retention of biological activity. A similar possibility may exist with regard to other oxidation-resistant muteins such as IFN-Beta. In addition, a substitution of a conserative amino acid such as alanine or serine at position 17 of the IFN-Beta mutein may made to confer additional stability on the mutein, by replacing cysteine residues which are not essential to the biological activity of the mutein. Any permutation of any of these changes alone or in combination with one or more other of these changes may be made to the oxidation-resistant muteins herein. For example, the following muteins are within the scope of this invention: $ser_{17}ala_{62}$IFN-Beta, $ala_{62}$IFN-Beta, $ser_{17}val_{62}$IFN-Beta, $val_{62}$IFN-Beta, $ser_{17}leu_{62}$IFN-Beta, $leu_{62}$IFN-Beta.

Applicants have deposited the following cultures with the American Type Culture Collection, Rockville, Maryland, USA (ATCC):

| Strain | ATCC Deposit No. | Deposit No. |
|---|---|---|
| pTRP3 | 39,946 | 18th Dec 1984 |
| pAW207 | 67,138 | 19th June 1986 |
| DG98 | 39,768 | 13th July 1984 |

These deposits were made under the provisions of the Budapest Treaty on the International Recognition of the Deposit of Microorganisms for the Purposes of Patent Procedure and the Regulations thereunder (Budapest Treaty). This assures maintenance of a viable culture for 30 years from date of deposit. The organisms will be made available by ATCC under the terms of the Budapest Treaty, and subject to an agreement between applicants and ATCC, which assures permanent and unrestricted availability of the progeny of the cultures to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638). The assignee of the present application has agreed that if the cultures on deposit should die or be lost or destroyed when cultivated under suitable conditions, they will be promptly replaced on notification with the viable specimen of the same culture. Availability of the deposited strains is not to be construed as a license to practice the invention in contravention of the rights granted under the authority of any government in accordance with its patent laws.

### Claims

1. An oxidation-resistant interferon-beta (IFN-Beta) mutein having substantially the same biological activity as a reference IFN-Beta protein and in which each methionine residue of the reference protein susceptible to chloramine T or peroxide oxidation is replaced by a conservative amino acid and wherein additional, non-susceptible methionine residues are not so replaced.

2. A mutein according to claim 1, wherein the conservative amino acid is glycine, alanine, serine, threonine, valine, isoleucine, leucine, asparagine, glutamine, glutamate, tyrosine, or phenylalanine.

3. A mutein according to claim 1, which is
   $ala_{62}$IFN-$\beta$, $ser_{17}ala_{62}$IFN-$\beta$,
   $leu_{62}$IFN-$\beta$, $ser_{17}leu_{62}$IFN-$\beta$,
   $val_{62}$IFN-$\beta$, or $ser_{17}val_{62}$IFN-$\beta$.

4. A pharmaceutical or veterinary formulation comprising a mutein according to any one of claims 1 to 3 formulated for pharmaceutical or veterinary use, and optionally an inert, non-allergenic, compatible carrier.

5. A method for preparing an oxidation resistant mutein of a biologically active IFN-Beta protein comprising substituting a conservative amino acid for each methionine residue susceptible to oxidation in said protein and wherein additional, non-susceptible methionine residues are not so-substituted.

6. A method according to claim 5 and further defined by the specific feature of claim 2 or wherein the product mutein is one defined in claim 3.

7. A recombinant DNA sequence which codes for an oxidation-resistant mutein according to any one of claims 1 to 3.

8. A recombinant expression vector comprising a DNA sequence according to claim 7 operably linked to control sequences compatible with a chosen host.

9. A chosen host cell transformed with an expression vector according to claim 8.

10. A method of producing a mutein according to any one of claims 1 to 3 comprising culturing cells of a chosen host transformed with a recombinant expression vector according to claim 8.

**Revendications**

1. Mutéine d'interféron-bêta (IFN-$\beta$) résistante à l'oxydation, ayant pratiquement la même activité biologique qu'une protéine d'IFN-$\beta$ de référence, et dans laquelle chaque reste méthionine de la protéine de référence, sensible à l'oxydation par la chloramine T ou par un peroxyde, est remplacé par un aminoacide conservateur, et dans laquelle les restes méthionine supplémentaires non sensibles ne sont pas ainsi remplacés.

2. Mutéine selon la revendication 1, dans laquelle l'aminoacide conservateur est la glycine, l'alanine, la sérine, la thréonine, la valine, l'isoleucine, la leucine, l'asparagine, la glutamine, le glutamate, la tyrosine ou la phénylalanine.

3. Mutéine selon la revendication 1, qui est:
   $ala_{62}$IFN-$\beta$, $ser_{17}ala_{62}$IFN-$\beta$,
   $leu_{62}$IFN-$\beta$, $ser_{17}leu_{62}$IFN-$\beta$,
   $val_{62}$IFN-$\beta$ ou $ser_{17}val_{62}$IFN-$\beta$.

4. Composition pharmaceutique ou vétérinaire comprenant une mutéine selon l'une quelconque des revendications 1 à 3, formulée pour une utilisation pharmaceutique ou vétérinaire, et éventuellement un véhicule inerte, compatible, non allergénique.

5. Procédé pour la préparation d'une mutéine, résistante à l'oxydation, d'une protéine d' IFN-$\beta$ biologiquement active, comprenant le remplacement par un aminoacide conservateur de chaque reste méthionine sensible à l'oxydation dans ladite protéine, et dans laquelle les restes méthionine supplémentaires non sensibles ne sont pas ainsi remplacés.

6. Procédé selon la revendication 5 et défini en outre par la caractéristique spécifique de la revendication 2 ou dans lequel la mutéine produite est une mutéine définie dans la revendication 3.

7. Séquence d'ADN recombinant qui code pour une mutéine résistante à l'oxydation selon l'une quelconque des revendications 1 à 3.

8. Vecteur d'expression recombinant comprenant une séquence d'ADN selon la revendication 7, fonctionnellement liée à des séquences régulatrices compatibles avec un hôte choisi.

9. Cellule d'hôte choisi, transformée par un vecteur d'expression selon la revendication 8.

EP 0 260 350 B1

10. Procédé de production d'une mutéine selon l'une quelconque des revendications 1 à 3, comprenant la culture de cellules d'un hôte choisi transformé par un vecteur d'expression recombinant selon la revendication 8.

**Patentansprüche**

1. Oxidations-resistentes Interferon-beta (IFN-beta)-Mutein, das im wesentlichen die gleiche biologische Aktivität wie ein Referenz-IFN-beta-Protein aufweist, und in dem jeder gegenüber Chloramin T oder für Peroxid-Oxidation empfindlicher Methioninrest des Referenzproteins durch eine konservative Aminosäure ersetzt ist, und in dem weitere, nicht-empfindliche Methioninreste nicht derart ersetzt sind.

2. Mutein nach Anspruch 1, wobei die konservative Aminosäure Glycin, Alanin, Serin, Threonin, Valin, Isoleucin, Leucin, Asparagin, Glutamin, Glutamat, Tyrosin oder Phenylalanin ist.

3. Mutein nach Anspruch 1, das
   $ala_{62}$IFN-$\beta$, $ser_{17}ala_{62}$IFN-$\beta$,
   $leu_{62}$IFN-$\beta$, $ser_{17}leu_{62}$IFN-$\beta$,
   $val_{62}$IFN-$\beta$, oder $ser_{17}val_{62}$IFN-$\beta$
   ist.

4. Pharmazeutische oder tiermedizinische Formulierung, enthaltend ein Mutein nach einem der Ansprüche 1 bis 3, die für eine pharmazeutische oder tiermedizinische Verwendung formuliert ist, und gegebenenfalls einen inerten, nicht-allergenen kompatiblen Träger.

5. Verfahren zur Herstellung eines Oxidations-resistenten Muteins eines biologisch aktiven IFN-beta-Proteins, wobei man jeden für Oxidation empfindlichen Methioninrest im Protein durch eine konservative Aminosäure ersetzt und wobei weitere, nicht-empfindliche Methioninreste nicht derart ersetzt sind.

6. Verfahren nach Anspruch 5 und ferner gekennzeichnet durch das bestimmte Merkmal von Anspruch 2 oder wobei das Muteinprodukt ein wie in Anspruch 3 definiertes ist.

7. Rekombinante DNA-Sequenz, die ein Oxidations-resistentes Mutein nach einem der Ansprüche 1 bis 3 codiert.

8. Rekombinanter Expressionsvektor, enthaltend eine DNA-Sequenz nach Anspruch 7, die mit für den gewählten Wirt kompatiblen Kontrollsequenzen funktionell verbunden ist.

9. Ausgewählte Wirtszelle, die mit einem Expressionsvektor nach Anspruch 8 transformiert ist.

10. Verfahren zur Herstellung eines Muteins nach einem der Ansprüche 1 bis 3, wobei man Zellen eines gewählten Wirtes züchtet, der mit einem rekombinanten Expressionsvektor nach Anspruch 8 transformiert ist.

14

MetSerTyrAsnLeu LeuGlyPheLeuGln ArgSerSerAsnPhe GlnCysGlnLysLeu
LeuTrpGlnLeuAsn GlyArgLeuGluTyr CysLeuLysAspArg MetAsnPheAspIle
ProGluGluIleLys GlnLeuGlnGlnPhe GlnLysGluAspAla AlaLeuThrIleTyr
GluMetLeuGlnAsn IlePheAlaIlePhe ArgGlnAspSerSer SerThrGlyTrpAsn
GluThrIleValGlu AsnLeuLeuAlaAsn ValTyrHisGlnIle AsnHisLeuLysThr
ValLeuGluGluLys LeuGluLysGluAsp PheThrArgGlyLys LeuMetSerSerLeu
HisLeuLysArgTyr TyrGlyArgIleLeu HisTyrLeuLysAla LysGluTyrSerHis
CysAlaTrpThrIle ValArgValGluIle LeuArgAsnPheTyr PheIleAsnArgLeu
ThrGlyTyrLeuArg Asn---

MATURE NATIVE

IFN-B AMINO ACID SEQUENCE

EP 0 260 350 B1